# EUROPEAN PATENT SPECIFICATION

(11) **EP 1 395 245 B1**
(45) Date of publication and mention of the grant of the patent: **07.03.2012**
(21) Application number: 02737110.3
(22) Date of filing: 24.05.2002
(51) Int. Cl.: A61L 27/10, A61L 27/52, A61L 27/56, C03C 1/00, C03C 3/06, C03C 3/078, C03C 3/097, C03C 4/00, C03C 11/00

(54) **BIOACTIVE FOAMED SOL-GEL AND METHOD OF MANUFACTURING THE SAME**
BIOACTIVES GESCHÄUMTES SOL-GEL UND DESSEN HERSTELLUNG
SOL-GEL BIOACTIVE EXPANSE ET SON PROCEDE DE FABRICATION

(30) Priority: 25.05.2001 US 293774 P
(43) Date of publication of application: 10.03.2004
(73) Proprietor: Imperial College Innovations, London SW7 2QA (GB); Sepulveda, Pilar, Swindon SN2 1ET (GB)
(72) Inventor: HENCH, Larry, L., London (GB); SEPULVEDA, Pilar, c/o Systems Level Technologies, Swindon SN2 1ET (GB)
(74) Representative: Wood, Jonathan Paul
(86) International application number: PCT/US2002/016319
(87) International publication number: WO 2002/096391

(56) References cited:
- WO-A1-91/04951
- WO-A2-98/29350
- US-A- 4 185 147
- US-A- 5 268 199
- US-A- 5 439 624
- US-A- 5 780 004
- DATABASE STN CHEMICAL ABSTRACTS, X [Online] 15 December 1997 (1997-12-15), NAKANISHI ET AL: "Preparation of double-pore silica by the alkoxide-based process" XP002067212 retrieved from CHEMICAL
- KAJI H ET AL: "Formation of porous gel morphology by phase separation in gelling alkoxy-derived silica: Affinity between silica polymers and solvent" JOURNAL OF NON-CRYSTALLINE SOLIDS, NORTH-HOLLAND PHYSICS PUBLISHING. AMSTERDAM, NL, vol. 181, no. 1-2, 1 February 1995 (1995-02-01), pages 16-26, XP004068043 ISSN: 0022-3093
- NAKANISHI K ET AL: "FORMATION OF HIERARCHICAL PORE STRUCTURE IN SILICA GEL" JOURNAL OF SOL-GEL SCIENCE AND TECHNOLOGY, SPRINGER, NEW YORK, NY, US, vol. 17, no. 3, 1 March 2000 (2000-03-01), pages 191-210, XP000947911 ISSN: 0928-0707
- CHEMICAL ABSTRACTS, AMERICAN CHEMICAL SOCIETY, US, 14 October 1996 (1996-10-14), XP000661944 ISSN: 0009-2258
- GILL I ET AL: "Bioencapsulation within synthetic polymers (Part 1): sol-gel encapsulated biologicals" TRENDS IN BIOTECHNOLOGY, ELSEVIER PUBLICATIONS, CAMBRIDGE, GB, vol. 18, no. 7, 1 July 2000 (2000-07-01), pages 282-296, XP004908534 ISSN: 0167-7799

## Description

### FIELD OF THE INVENTION

The invention relates to a process for making foamed glasses and ceramics from sol-gels. The invention further relates to porous substrates for cell growth or other tissue engineering uses.

### BACKGROUND OF THE INVENTION

A variety of methods for producing porous ceramics have been developed. However, there remains a need in the art to provide a process for producing macroporous bioactive glasses and ceramics from a sol-gel process.

### SUMMARY OF THE INVENTION

Given the need for suitable macroporous scaffolds in tissue engineering, and the advantageous properties of bioactive glasses, the present invention relates to a method for producing macroporous foamed glasses and ceramics, including compositions within the bioactivity range of silica-based glasses, by combining a sol-gel process and a foaming process.

In one aspect of the invention, a process is provided for producing foamed sol-gel compositions comprising hydrolyzing a reaction mixture capable of forming a sol-gel; adding a catalyst to the reaction mixture to accelerate condensation of the reaction mixture; foaming the reaction mixture with a surfactant with vigorous agitation until the reaction mixture begins to gel; casting the foamed reaction mixture into a mold of desired shape to obtain the foamed sol-gel; and ageing, drying and thermally stabilizing the foamed sol-gel.

In another aspect of the invention, a process is provided for producing a foamed bioactive sol-gel with a hierarchical structure having macropores with a mean size of 10 to 500 micrometers and mesopores of 10 to 500 angstroms comprising hydrolyzing a reaction mixture comprising metal alkoxides capable of forming a bioactive sol-gel; accelerating condensation of the reaction mixture by adding an acidic catalyst; foaming the reaction mixture by adding a surfactant and vigorously agitating the reaction mixture; casting the foamed reaction mixture into a mold of desired shape to complete formation of the foamed bioactive sol-gel; and ageing, drying and thermally stabilizing the foamed bioactive sol-gel.

The foamed sol-gels of the invention may be useful as tissue engineering scaffolds or substrates. These scaffolds may be used, for example, for growing cells, such as osteoblasts to form a bone implant in-vitro for ultimate implantation into humans, or for bone grafts. Additionally, the foamed sol-gels may find use as biological filtration devices, such as for protein separation or bacterial filtration, or may be used as drug delivery devices by adsorbing drugs into the foamed sol-gel structure.

In one aspect of the invention, a biocompatible substrate for growing cells is provided comprising a foamed sol-gel having a hierarchical structure, macropores with a mean size of 10 to 500 micrometers and mesopores in the range of 10 to 500 angstroms.

In another aspect of the invention, a biological filter is provided comprising a bioactive foamed sol-gel having a three-dimensional open network of spherical pores that are thoroughly interconnected, macropores with a mean size of 10 to 500 micrometers and mesopores in the range of 10 to 500 angstroms, and wherein the bioactive foamed sol-gel is formed from binary SiO₂-CaO glass or ternary SiO₂-CaO-P₂O₅ glass.

In a further aspect of the invention, a drug delivery device is provided comprising a bioactive foamed sol-gel having a three-dimensional open network of spherical pores that are thoroughly interconnected, macropores with a mean size of 10 to 500 micrometers and mesopores in the range of 10 to 500 angstroms and wherein the bioactive foamed sol-gel is formed from binary SiO₂-CaO glass or ternary SiO₂-CaO-P₂O₅ glass.

In an additional aspect of the invention, a biocompatible substrate useful in tissue engineering structures is provided comprising a foamed sol-gel with macropores with a mean size of 10 to 500 micrometers and mesopores in the range of 10 to 500 angstroms formed by first foaming a silica-based sol-gel in the presence of a catalyst and a surfactant with vigorous agitation at a controlled temperature of 22 to 28°C and then casting the foamed silica-based sol-gel into a mold of desired shape.

### BRIEF DESCRIPTION OF THE DRAWINGS

Figure 1 illustrates a schematic diagram of a process for foaming sol-gel in accordance with the present invention.

Figure 2 illustrates the structure obtained by foaming 58S sol in accordance with the present invention after thermal stabilization at 800°C.

Figure 3 illustrates the structure obtained by foaming SiO₂ in accordance with the present invention after thermal stabilization at 600°C.

Figure 4 illustrates pore size distribution of 58S at various levels of foaming.

Figure 5 illustrates the structure obtained by foaming 70S30C sols at various levels of foaming.

Figure 6A is a graph illustrating the gelling time as a function of foaming temperature (T_{f}) as discussed in Example 5.

Figure 6B is a graph illustrating the foam volume as a function of foaming temperature as discussed in Example 5.

Figure 7 is a graph illustrating pore size distributions as discussed in Example 5.

Figure 8A illustrates an SEM micrograph of a scaffold with a crack-free pore network as discussed in Example 5.

Figure 8B illustrates an SEM micrograph of a scaffold as discussed in Example 5.

Figure 9 is a graph illustrating gelling time as a function of gelling agent concentration as discussed in Example 5.

Figure 10 is a graph illustrating foam volume as a function of added water concentration.

### DETAILED DESCRIPTION OF THE INVENTION

The present invention provides a novel method for producing foamed sol-gel glasses and ceramics. The method combines a sol-gel technique with foaming to incorporate macropores (herein meaning pores in the micron-millimeter range) into a material that is typically known to be mesoporous (herein meaning pores in the range of 10-500 angstroms) without adversely affecting the bioactivity of the materials and maintaining structural integrity as in, for example, a monolithic shape. The invention comprises providing a mixture of reactants capable of forming a sol-gel, such as, for example, a mixture of metal alkoxides, preferably capable of forming a bioactive sol-gel. A foam is produced from the liquid sol, rather than a particulate or colloidal suspension of particles, preferably by the addition of surfactants and agitation to entrap air. Condensation of the foam is catalyzed to promote fast setting of the foam into a gelled body, preferably by the addition of an acid or base. The gelled bodies may then be aged, dried, and thermally treated to stabilize the structure. The resulting macroporous structure typically is characterized by a three-dimensional open network of spherical pores (formed from the bubbles in the foam) that are thoroughly interconnected. The structure may also be interconnected with continuous channels. The foamed sol-gels preferably exhibit a hierarchical structure.

The resulting structure preferably has pores with a mean size of 10-500 micrometers and mesopores in the range of 10-500 angstroms. In one preferred aspect of the invention, the resulting foamed sol-gel has pores with a mean size of 50 to 250 micrometers and mesopores in the range of 75 to 250 angstroms. The resulting material preferably is formed from a silica-based sol-gel and may comprise a silica-based glass (such as pure silica glass (SiO₂) or glasses in the binary SiO₂-CaO or ternary SiO₂-CaO-P₂O₅ systems), and may also comprise a ceramic (such as sol-gel derived mullite or zirconia). Preferably, the material will have a composition of from 40% to 100% SiO₂, 0% to 40% CaO, 0% to 15% P₂O₅, and 0% to 15% Na₂O. Other trace elements may also be present such as Ag, Zn, Cu, and Mg, among others. Bioactive silica-based compositions are known and typically are capable of forming hydroxycarbonate apatite when exposed to physiological fluids.

The foamed sol-gel material preferably comprises biocompatible compositions or substrates useful for tissue engineering scaffolds for healing tissue defects and as bone grafts for aiding bone regeneration and repair. Such scaffolds, prepared as foamed sol-gels by the process of the present invention, typically include an interconnected network with macropores to enable tissue ingrowth and nutrient delivery to the center of regenerating tissue and mesopores to promote cell adhesion. Preferably, the foamed sol-gel scaffold will resorb at controlled rates to match that of tissue repair and be made from a processing technique that can produce irregular shapes to match that of the defect in the bone of the patient.

Other methods of producing porous ceramics have been explored. Reviews of the most common techniques are described, for example, in Sepulveda (Ceram. Bull, 76 [10] 61-65, 1997) and Saggio-Woyansky (Am. Ceram. Soc. Bull. 71 [11] 1674-1682, 1992). Porous ceramics provide unique properties and structures that allow a wide range of applications, distinguished according to the pore size range and the material composition. Pores may vary in size from as small as the nanometer range typical of sol-gel derived materials (see, e.g., U.S. Pat. No. 6,010,713) up to a few hundred micrometers or even a few millimeters in diameter as found in foamed or reticulated materials (see, e.g., PTY Patent Application 1321093 "Foamed Ceramic Material" 1970 and Int. Pat. WO93/04013 "Porous Articles" 1993).

Macroporous structures that mimic polymeric foams have been widely explored. Some examples are illustrated in the following articles: Powell, S. J., Evans, J. R. G. "The structure of ceramic foams prepared from polyurethane--ceramic suspensions", Mat. Manufact. Processes, 10 [4] 757-771, 1995 and Paiva, A. E. M., Sepulveda, P., Pandolfelli, V. C. "Processing and thermomechanical evaluation of fibre-reinforced alumina filters", J. Mat. Sci., 34 2641-2649, 1999. One method for producing such a structure comprises dipping a polymeric sponge into a suspension of ceramic particles to form a thin layer of ceramic coating, then drying and eliminating the polymer at higher temperatures, thereby leaving a replica.

A method to produce cellular foams from a preceramic polymer (a silicone resin) and reagents to produce blown polyurethane is described in Colombo, P., Modesti, M. "Silicon oxycarbide foams from a silicone preceramic polymer and polyurethane", J. Sol-gel Sci. Tech., 14 [1] 103-111, 1999. After pyrolysis, polymer-to-ceramic transitions occur, forming amorphous silicon oxycarbide (SiOC). Another example of ceramic foam manufacturing involves the generation of gas by heat treatment of crystals of the aluminum chloride isopropyl ether complex (see, e.g., Grader, G.S., Shter, G.E., and deHazan,Y. "Novel ceramic foams from crystals of AlCl3((Pr2O)-O-i) complex", J. Mat. Res., 14 [4] 1485-1494, 1999).

Highly porous ceramics have been produced from foaming to obtain pores in the range of 20 µm up to 1-2 mm. For this process, fluid processing, mostly from particulate suspensions or colloidal suspensions can be used. Bubbles may be incorporated by bubbling air through the fluid medium, agitation, blowing agents, evaporation of compounds, or reactions that produce gas. The main difficulty in the production of porous bodies through foaming is the consolidation stage, regardless of whether it involves drying, setting by binder addition, transition of suspensions into a semi-solid body, or any other means.

In the manufacture of foams, the stabilization of bubbles is accomplished with the help of specific surfactants. This step is very important to ensure the manufacture of uniform foams. The films that surround the bubbles in the liquid foam must remain stable until the structure solidifies. The science of surfactants is very extensive. Surfactants are macromolecules composed of two parts, one hydrophobic and one hydrophilic. Due to this configuration, surfactants tend to adsorb onto gas-liquid interfaces with the hydrophobic part being expelled from the solvent and a hydrophilic part remaining in contact with the liquid. This behavior lowers the surface tension of the gas-liquid interfaces, making the foam films (which would otherwise collapse in the absence of surfactant) thermodynamically stable.

An illustration is the uniform open- or closed-celled ceramics obtained from foaming aqueous particulate suspensions and gelcasting (described, e.g., in Sepulveda, P., Binner., J.G.P. "Processing of cellular ceramics by foaming and in situ polymerization of organic monomers", J. Eur. Ceram. Soc., 19 [12] 2059-66, 1999). The method includes foaming of suspensions of ceramic particles, and in situ polymerization of previously incorporated organic monomers to set the liquid foam into a semi-dried body. Non-ionic surfactants in various concentrations were used to stabilize the foam and to allow different foam volumes to be raised to vary the density. Non-toxic acrylic monomers and dimers were used for polymerization. This procedure was shown to enhance the matrix microstructure and mechanical properties, due to the strong crosslinked polymeric network formed during polymerization. Alumina, zirconia, mullite, cordierite, and hydroxyapatite were produced. Foaming at various levels allowed the variation of pore size and subsequently a wide range of permeability, as shown by Innocentini, M.D.M., Sepulveda, P., Salvini, V. R., and Pandolfelli, V. C. "Permeability and structure of cellular ceramics: a comparison between two preparation techniques", J. Am. Ceram. Soc., 81 [12] 3349-52, 1998.

Methods in the art that report making cellular inorganic materials by applying the foaming concepts to sol-gel systems are scarce, since the complexities of the sol-gel process already impose a limitation towards the production of monoliths. In the sol-gel method, typically organo-metallic precursors as liquids are mixed, sometimes with oxides in multi-component systems. The solution thus formed (sol) from these organic precursors is sensitive to changes in pH, temperature and water content. Altering any of these properties causes the mixture to begin to polymerize from solution to form a gel (hence the term sol-gel). Once the gel forms, processing in the form of holding the mixture at elevated temperatures causes the mixture to condense. At high processing temperatures, the organic phase is volitalized and the inorganic phase is left.

Illustrations of work in this field include Wu et al. (J. Non-Cryst. Solids, 121, 407-412, 1990) and Fujiu et al. (J. Am. Ceram. Soc., 73 [1] 85-90, 1990). The method reported employs viscosity control during sol-to-gel transition to stabilize bubbles generated from freon droplets dispersed in the sol. Silica sol, a mixture of aqueous boehmite and silica sol, and zirconia sol (zirconia nitrate) were used as starting reagents to produce foamed silica, foamed mullite, and foamed zirconia, respectively. Sodium dodecyl sulfate and 1-dodecanol were added as surfactants for foam stabilization. The viscosity control was obtained by adjustment of pH with H₂SO₄. Freon, whose boiling point is 23.8°C, was added and incubation was carried out at temperatures above the boiling temperature for bubble formation. This step was controlled to occur simultaneously with gelation.

These methods describe combinations of using silica sols with other particulates in order to obtain foamed structures. They do not disclose producing foams from sol-gels alone as the particulates act to stabilize the foamed structure. The situation is more complex when trying to form a foam from any sol-gel material.

In biomedical applications, there is a large need for macroporous structures that may be used as matrixes for tissue engineering and as devices for bone repair. The open channels that connect the three-dimensional network allow tissue in-growth both in vivo or in cultures prior to implantation, accelerating healing and tissue regeneration and allowing the use of the patient's own cells. Bone grafts from processed corals, allograph or xenograph bone or porous hydroxyapatite have offered good alternatives for bone implants. A review of porous implant structure is given in U.S. Pat. No. 6,063,117. The use of foams for bone substitute material produced from a carbon foam infiltrated by chemical vapor deposition (CVD) mimicking the microstructure of natural cancellous bone is described in U.S. Pat. No. 5,282,861. However, carbon foams are not bioactive. Foaming a ceramic suspension from particulated hydroxyapatite has shown that materials are suitable for bone grafting since organic reagents used in the processing are completely eliminated without leaving residue (see, e.g., Sepulveda, P., Binner, J.G.P., Rogero, S.O., Higa, O.Z., Bressiani, J.C. "Production of porous hydroxyapatite by the gel-casting of foams and cytotoxic evaluation", J. Biomed. Mater. Res. 50 pp. 27-34, 2000 and Sepulveda, P., Bressiani, A.H., Bressiani, J. C., Konig Jr., B. "In vivo evaluation of hydroxyapatite foams"). These materials begin with particulates, not from the alkoxide sol. The sol-gel process typically leads to porous materials with pores in the range of about 10-2000 angstroms, as indicated by U.S. Pat. No. 4,810,674 and U.S. Pat. No. 4,849,378. Porous bioactive sol-gel compositions have been disclosed in U.S. Patent No. 6,010,713 for treating orthopaedic defects. The use of compositions that are bioactive has been preferred to less bioactive ones because of the rapid healing caused by an interfacial bond with surrounding tissue through a series of chemical reactions. Bioactive glasses are known to the art, and typically contain less than 60 mole percent of SiO₂, may have a high sodium and calcium content (20-30% each), and a high molar ratio (i.e., ~5) of calcium to phosphorus. Binary compounds in a wide range of SiO₂-CaO ratios have also been shown to be bioactive (see, e.g., Saravanapavan, P., Hench, L. L. "Low temperature synthesis and bioactivity of gel-derived glasses in the binary CaO-SiO₂ system" submitted to the J. Biomed. Mat. Res.). These materials achieve their bioactivity by releasing ions from the glass into solution, with the subsequent formation of a hydroxycarbonate apatite layer. It is this reactivity that is unique in these materials and have been extensively described. Forming a foam from bioactive particulates is not feasible because the process requires an aqueous solution, thus causing reactivity of the material in the processing. This will render the material non-bioactive.

None of the methods previously explored provide a novel method for producing foamed sol-gel glasses and ceramics with the advantages of the present method.

### Preparation of foamed sol-gel

Preparation of foamed sol-gel involves several steps, including the mixture of reagents (such as tetraethoxyorthosilicate (TEOS) (Si(OC₂H₅)₄), triethoxyphosphate (TEP) (OP(OC₂H₅)₃), and Ca(NO₃)₂•4H₂O), hydrolysis, foaming, and condensation leading to gelling. Both hydrolysis and condensation may be catalyzed by the addition of a variety of reagents including acids, bases, and other organic components. These catalyses alter the pore network and may also affect the crystalline structure of the material. Examples of possible foamed glasses are described below and include three silica-based systems: unary SiO₂, binary SiO₂-CaO (70:30 % mol), and ternary SiO₂-CaO-P₂O₅ (60:36:4 % mol).

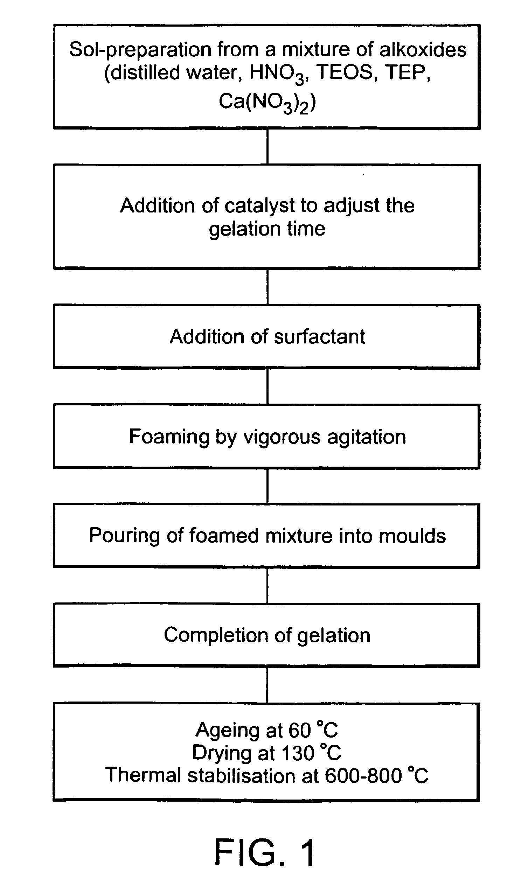

Figure 1 illustrates a schematic diagram of one aspect of a process for foaming sol-gel in accordance with the present invention. As shown in the figure, a sol preparation may be prepared from a mixture of alkoxides. Typically, these alkoxides are metal alkoxides such as tetraethoxyorthosilicate (TEOS) (Si(OC₂H₅)₄), and triethoxyphosphate (TEP) (OP(OC₂H₅)₃), but alkoxides of calcium, titanium zirconium, magnesium aluminum, iron and potassium, among others, may also be used. The reaction mixture for the sol preparation may include additional reactants of hydrolysis catalysts such as nitric acid, calcium nitrate, water or other reagents typically known for use in hydrolysis and sol preparation, or mixtures thereof.

After the addition of a catalyst to adjust the gelation time and a surfactant, the mixture is foamed by vigorous agitation. The catalyst may be an acid or base, preferably, a strong acid such as HF. The surfactant may be any surfactant known to produce foaming or mixtures thereof, preferably a non-ionic or anionic surfactant. Preferably, the foaming step will take place at a temperature of 22 to 28°C, particularly preferably when the catalyst is HF. The foamed mixture is then poured into molds where gelation is completed. The foamed sol-gel is then aged at elevated temperature, for example, 60°C., dried at elevated temperature, for example, 130°C., and thermally stabilized at 600-800°C.

The invention will now be more fully explained by the following examples. However, the scope of the invention is not intended to be limited to these examples.

### EXAMPLES OF SOL MIXTURE REAGENTS

For the preparation of pure silica, the reagents involved in the sol-gel preparation were mixed in the following order: distilled water (162 ml), 2N nitric acid (HNO₃) (27 ml), and tetraethoxy orthosilicate 99% purity (TEOS) (167 ml).
For the preparation of 58S (60% SiO₂, 36% CaO, 4% P₂O₅), the reagents involved in sol-gel preparation were mixed in the following order: distilled water (89.86 ml), 2N HNO₃ (14.94 ml), TEOS (122.70 ml), triethoxy phosphate (TEP) (12.52 ml), and calcium nitrate (77.98g).

For the preparation of 70% SiO₂-30% CaO, the reagents involved in sol-gel preparation were mixed in the following order: distilled water (100.80 ml), 2N HNO₃ (16.80 ml), TEOS (103.92 ml), and calcium nitrate (47.20 g).

The above solutions were all gently stirred with a magnetic stirrer for 1 hour for complete hydrolysis.

### Catalysts for condensation

Prior to foaming, a catalyst or gelling agent is preferably added to accelerate the condensation and consolidate the foam into a gel before collapse of bubbles takes place deteriorating the porous structure.

### EXAMPLES OF CATALYSTS

Two catalysts, HF and NH₄OH, were tested to accelerate and control the time for condensation. Preliminary tests were carried out to evaluate the time for gelation to start. The tests involved pouring 5 ml of hydrolyzed sol into cylinders and adding catalyst in various concentrations to observe the time for setting of the sol into a gel.

*HF(5%) catalyzed reactions:* Adding 0.25 ml of HF (5%) in 5 ml of sol caused gelation to take place within a period varying from 2-3 min, depending mostly on the room temperature. All three tested sol compositions (described above) behaved similarly, showing an onset for gelation to take place. This period is very important to allow the process of foaming to be conducted.

*NH₄OH catalyzed reactions:* Various concentrations of this catalyst were added in 5 ml silica sol. The concentrations tested either produced very fast and inhomogeneous gelation, in the form of lumps, or instant gelation without an onset. Lowering the concentration did not lead to gelation within a reasonable period of time. The tested concentrations and observations are listed in Table 1.

**Table 1: Amounts of NH₄OH added into 5 ml silica sol and description of gelation**

| NH₄OH Volume | NH₄OH Concentration | Description of the reaction |
|---|---|---|
| 0.25 ml | 33% | instant and localized gelling forming lumps |
| 0.25 ml | 5.5% | instant and localized gelling forming lumps |
| 0.25 ml | 3.0% | instant and localized gelling forming lumps |
| 1.00 ml | 2.1% | instant gelation forming a homogeneous gel |
| 0.50 ml | 2.1% | instant gelation forming a homogeneous gel |
| 0.25 ml | 2.1% | did not gel within 30 min |
| 0.25 ml | 1.57% | did not gel within 30 min |

Due to the difficulty in controlling gelation and achieving an onset for gelation with NH₄OH, HF was used for catalysis in the examples discussed below.

### Foaming of sol-gel systems:

Foaming is a fairly simple procedure; however, in silica sol-gel systems it may be difficult to achieve foaming since silica is an anti-foaming agent. Thus, the use of surfactants that lower the surface tension is not sufficient to guarantee the rise of a reasonable foam volume, at least 2 times the starting volume of sol, preferably 3 to 6 times the starting volume. A large variety of surfactants that are known to be good foamers can be used. The surfactants tested for foam generation include Tergitol TMN10 (Aldrich Co.), which is a non-ionic surfactant comprised of polyethylene glycol trimethylnonyl ether; Tergitol Foam 2X (same composition as Tergitol TMN10, but with different molecular range); Teepol^{®}, which is an anionic surfactant; combinations of sodium dodecyl sulfate and 1-dodecanol; and detergent containing 2% nonoxynol-9 and 0.5% PCMX (Day-Impex Ltd.). All examples given in this application make use of Tergitol TMN10 (Aldrich Co.) as the surfactant, because the suitable amount of foam produced ranged from 2 to 4 times the original sol volume. Varying the surfactant concentration influences the foam volume, which is an important factor that determines the density and connectivity of the macroporous structures.

### EXAMPLE OF FOAMING

The procedure of foaming was accomplished as follows: 50 ml of sol were transferred into a beaker along with 1 ml HF (5%). The sol was mixed and observed for changes in viscosity. After approximately 5 minutes, the surfactant (Tergitol TMN10) was added (1 ml) and the mixture was agitated at high speed using a double-blade mixer. (The addition of surfactant helps reduce the surface tension and allows easier foaming.) In order to obtain stable foam, the foaming procedure had to be carefully synchronized with the viscosity increase that results from rapid condensation. The mixture was foamed for 4-5 minutes until the foam volume started to rise more steeply and the viscosity was notably higher, which indicated the beginning of gelation. Stirring was then stopped and the foamed gel was rapidly poured into cylindrical or squared containers.

### Drying and ageing

The process of ageing and drying applied to the foams was typical of the sol-gel processing, and took an average of from 5-7 days.

The sealed containers were placed in an oven at 60°C. for 72 hours to promote ageing and strengthening of the gels. For the drying stage, the containers' caps were slightly opened to allow slow solvent evaporation. The drying cycle was accomplished in three steps: (1) 60°C for 20 hours, (2) 90°C for 24 hours, and (3) 130°C for 40 hours, at heating rates of 0.1 °C/min., carried out in sequence. It has been discovered that the long drying times are necessary to avoid collapse of the structures.

### Thermal Stabilization

Thermal stabilization of foamed bodies was carried out by applying the following heating cycle: heating at 10°C/min up to 100°C, heating at 0. 5°C/ min up to 300°C, holding at this temperature for 2 hours, heating at 1°C/min up to 600°C, holding at this temperature for 5 hours, and cooling at a rate of 5°C/min down to room temperature. A few bodies were treated at higher temperatures in order to vary the matrix densification degree. The higher stabilization temperatures resulted in a more dense structure which gave a higher mechanical strength, but lowered the ionic reactivity, hence lowered the bioactivity of these samples.

At the end of the process, monolithic samples were obtained with a diameter of approximately 25 mm in various shapes. The results show that the process is very versatile and can be adapted to produce any shape as long appropriate molds are used.

### EXAMPLES OF FOAMING VARIOUS COMPOSITIONS

The following examples describe a series of tests carried out to foam various compositions of sol. The foaming procedure used was the same as described above.

### EXAMPLE 1: Foaming of silica sol

The following mixture was added into a beaker for foaming:
50 ml silica sol
1 ml HF (5%)
1 ml Tergitol TMN10

Foaming of the mixture was not successful at first. Little foam was generated. Thus, after 5 min agitation, an additional aliquot of 1 ml Tergitol was added. A volume of 200-300 ml foam was then produced. When the foam was firm and the viscosity started to increase, the agitation was stopped and the foamed sol was poured into the molds and sealed. Gelling took place 17-20 minutes after catalyst addition.

Other ratios of surfactant and catalyst were also employed which resulted in the rise of a reasonable amount of foam, as follows:
50 ml silica sol, 1.5 ml HF, 0.3 ml Tergitol TMN10
100 ml silica sol, 3 ml HF, 1.5 ml Tergitol TMN10

### EXAMPLE 2: Foaming of 58S

The following mixture was added into a beaker for foaming:
100 ml 58S sol
3 ml HF
1.5 ml Tergitol TMN10

The mixture did not rise into a foam, even after 10 minutes of continuous agitation. Considering that more components were being introduced into the sol-gel system and that a lower water/TEOS ratio (R=8) was being used compared to that of silica sols (R=12), more distilled water was introduced to the sol mixture to enable easier foaming. This demonstrates the sensitivity of the ratio of components necessary to produce a stable foam from the starting sol. After this, foam was generated as previously noted for example 1.

Other ratios of surfactant and catalyst were employed for 58S, as follows:
(1) 50 ml sol, 10 ml water, 1 ml HF, 1 ml Tergitol TMN10. Foaming was accomplished and gelation took place within approximately 7 minutes after catalyst addition.
(2) 50 ml sol, 20 ml water, 1 ml HF, 1 ml Tergitol TMN10. Foaming was successful and gelation took place in approximately 10 minutes after catalyst addition.

In the production of foam from 58S sol, it was difficult to identify the exact time for pouring the gels into the containers. Early pouring, when the sol was not yet undergoing gelation, led to foam collapse and the deposit of a layer of liquid sol at the bottom of the container.

### EXAMPLE 3: Foaming of 70%SiO2-30%CaO (S70C30)

The following mixture was added into a beaker for foaming:
100 ml S70C30 sol
3 ml HF
1.5 ml Tergitol TMNI0

Agitation generated only a very small amount of foam in this mixture and gelation took place while stirring was still being carried out due to the difficulty in identifying viscosity variation. This resulted in non-uniform bodies being formed that cracked during drying and thermal stabilization.

Another ratio of surfactant and catalyst was employed as follows: 50 ml sol, 10 ml water, 1 ml HF, 1.5 ml Tergitol TMN10. Foaming was accomplished with success because of the further addition of water, although the time for pouring was still difficult to identify, as described above.

### EXAMPLE 4: Foaming of 70%SiO2-30%CaO (S70C30) with low shear rate agitation

The following mixture was added into a beaker for foaming:
20 ml of sol
0.5 ml HF
1 ml Tergitol TMN10

The mixture was placed in a sealed container and manually shaken to generate a foam. Agitation was discontinued when there was visual indication of the gelation, that is when the viscosity of the sol was significantly increased, generally from about 3 Pascal-seconds to about 15 Pascal-seconds.

### CHARACTERIZATION OF THE RESULTING POROUS STRUCTURE

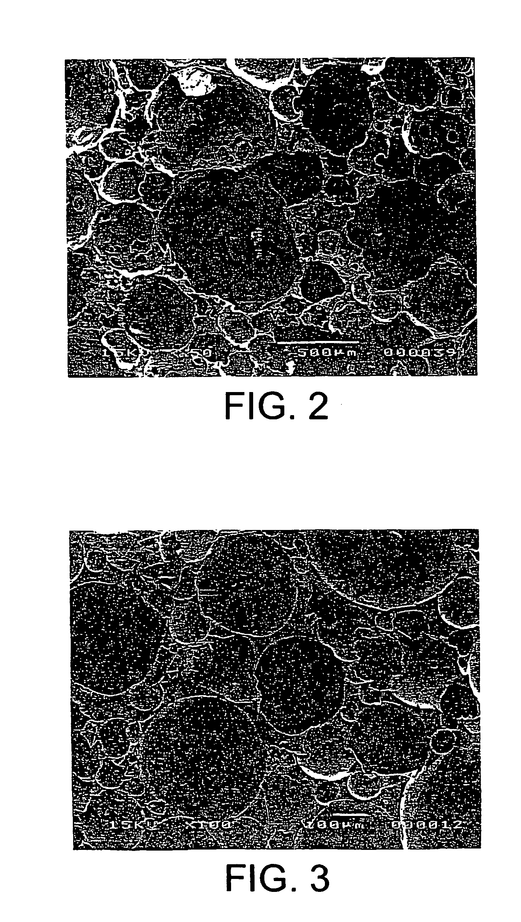

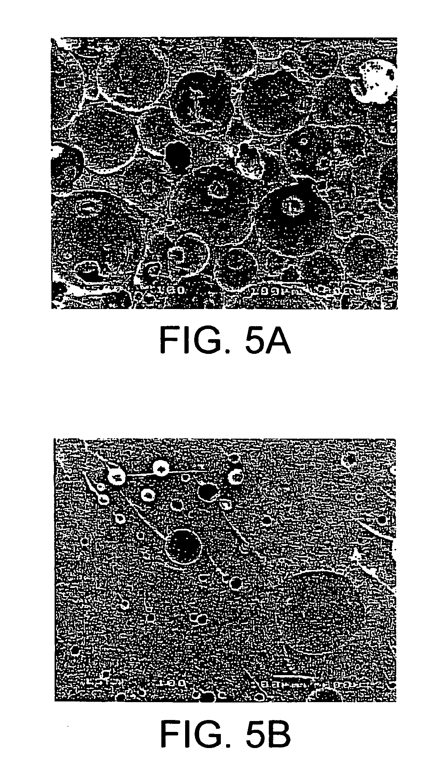

The micrographs shown in Figures 2, 3 and 5 illustrate the typical pore structure that is produced by foaming sol-gel systems. The larger pores (cells) are derived from the bubbles in the liquid foam and the smaller pores that connect the larger cells are derived from the rupture of bubble walls.

Thermal treatment variation may provide structures that vary in densification degree. Thus, dissolution rates and corresponding bioactivity levels of relevant glass compositions may be controlled through microstructural control.

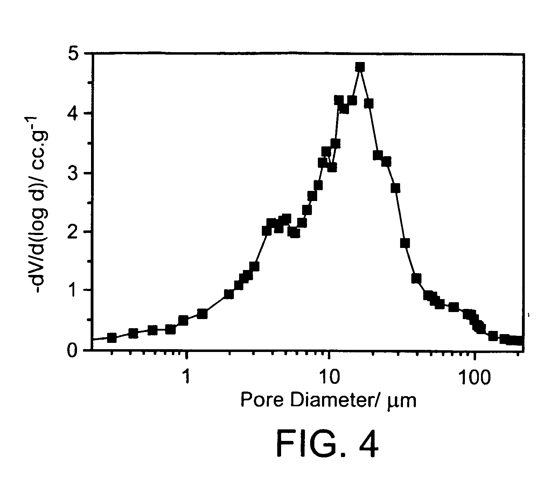

Figure 4 shows a typical mercury porosimetry curve, where a range of pore sizes can be detected within 10-100 µm. Even though Hg porosimetry is not able to measure pores larger than 200 µm, pores larger than 500 µm are clearly observed in the micrographs shown in Figures 2, 3 and 5. Depending on the amount of foam generated, different pore size ranges can be produced.

### EXAMPLE 5

Many factors in the methods taught herein may affect the structure and properties of the foamed sol-gel materials which may be manipulated to obtain specific architectures, to produce specific pore size ranges and controlled rates of glass dissolution. Such factors include the temperature at which the foaming process is carried out, surfactant type and concentration, gelling agent type and concentration, added water concentration and glass composition.

The effect of other factors on the ternary 58S (60 mol% SiO₂, 36 mol% CaO, 4 mol% P₂O₅) composition, which is believed to be the most bioactive of the sol-gel-derived bioactive glasses, were investigated.

The steps for manufacture are as described above, with the reactants mixed in stoichiometric proportions depending on the glass composition. The procedure was carried out using three compositions: pure silica SiO₂ (100S), the binary 70%SiO₂-30%CaO (70S30C), and the ternary (58S) systems, in molar percentage. Sol-gel precursors tetraethoxylorthosilicate (TEOS, Si(OC₂H₅)₄), triethoxyphosphate (TEP, OP(OC₂H₅)₃), and calcium nitrate Ca(NO₃)₂•4H₂O, were mixed in D.I. (deionised) water in the presence of 2N nitric acid (HNO₃), a catalyst for hydrolysis. Simultaneous hydrolysis and polycondensation reactions occur to begin formation of a silica network. Viscosity of the sol increases as the condensation reaction continues. On completion of hydrolysis, aliquots of 50 ml sol were foamed by vigorous agitation with the addition of 1.5 ml surfactant (Teepol^{®}, an anionic surfactant), D.I. water (believed to improve foamability of surfactant) and HF (catalyst for polycondensation). The surfactant stabilizes the bubbles that are formed by air entrapment during the early stages of foaming. As viscosity rapidly increased and the gelling point was approached the solution was cast into airtight molds. The gelation process provides permanent stabilization for the bubbles. The samples were then aged at 60°C for 72h, dried at 130°C for 48h and thermally stabilized at 600°C for 22h, according to established procedures. The temperature, concentration of added water and concentration of HF in the solution, were varied independently to obtain specimens of different porosities. Foaming temperature was thermostatically controlled by a water bath. At least three separate batches were produced for each variable to ensure reproducibility. The resulting foams were characterized using scanning electron microscopy (JEOL, JSM T220A), mercury porosimetry (PoreMaster 33, Quantachrome) and nitrogen adsorption (Autosorb AS6, QuantaChrome) to measure macro and mesopore size distributions respectively. B.E.T. analysis was used to determine the specific surface area. The pore diameter distribution was calculated by the BJH method applied to the N₂ desorption curves. The types of isotherms were evaluated according to their shape and type of hysteresis between adsorption-desorption modes.

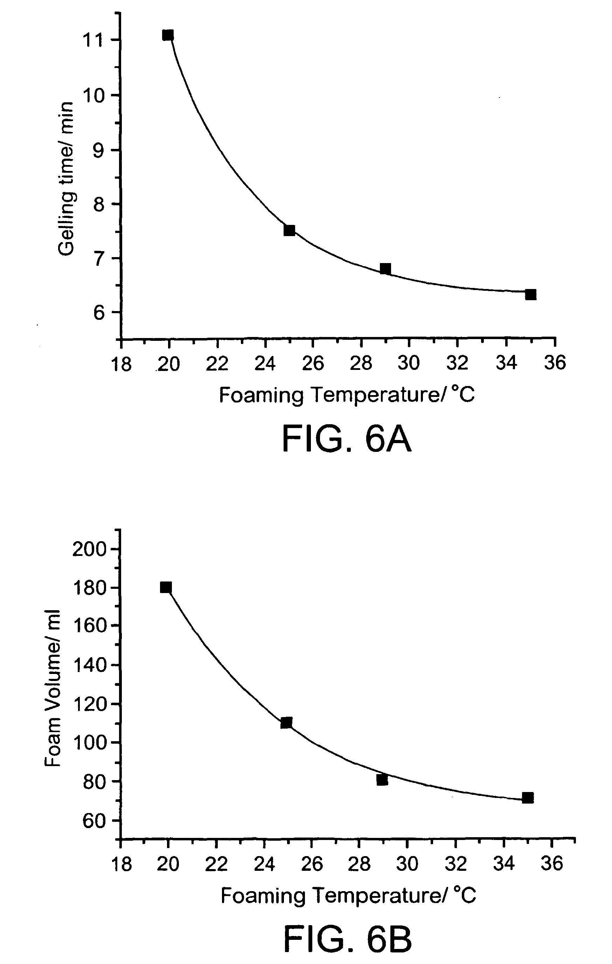

Figure 6A shows a graph of gelling time as a function of foaming temperature (T_{f}), where foaming temperature is defined as the temperature of the water bath in which the sol was foamed. The graph shows that as foaming temperature was increased from 20°C to 35°C, the gelling time decreased from 11 min 10s to 6 min 20s. All gelling times were reproducible with a 5% range. The gelling time is believed to have decreased because the condensation rate increased as a result of the increase in temperature. Foam volume as a function of foaming temperature followed a similar relationship, as shown in Figure 6B, decreasing from approximately 180 ml at 20°C to 70 ml at 35°C. The foam volumes were reproducible within 10 ml for all temperatures, except at 20°C, where foam volumes greater than 200 ml were produced. For foam volumes above 180 ml, the bubbles produced are so large that the polycondensation reaction cannot stabilize them and the foams collapse.

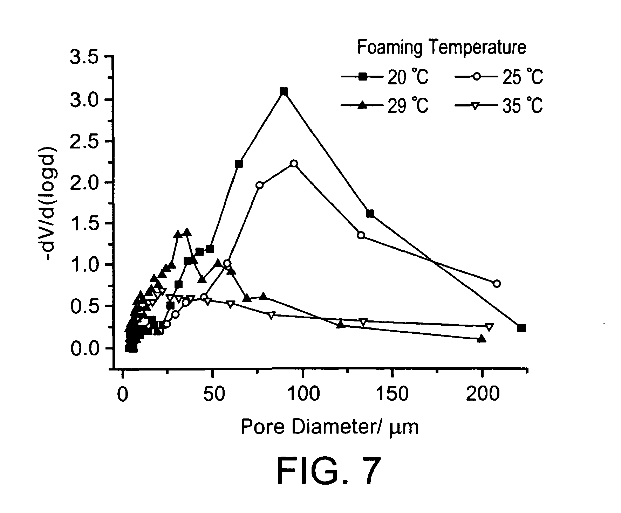

Figure 7 shows pore distributions, attained by mercury porosimetry from foams produced using foaming temperatures of 20°C, 25°C, 29°C, and 35°C. All pore distributions were wide, implying that foams produced at each of the four temperatures contained some pores greater than 200µm (limit of the mercury porosimeter). Scaffolds foamed at temperatures of 20°C and 25°C, exhibited approximately normal pore distributions, with modal pore diameters of 95 µm. Above 25°C pore distributions followed a positive skew and the amount of skew increased as temperature increased, with a modal pore diameter of approximately 35 µm at 29°C and 22 µm at 35°C. The pore distributions were not homogeneous, therefore the mode of the pore distribution is of most interest when initially characterizing the foams, as the mode represents the pore size that is most frequent in the sample.

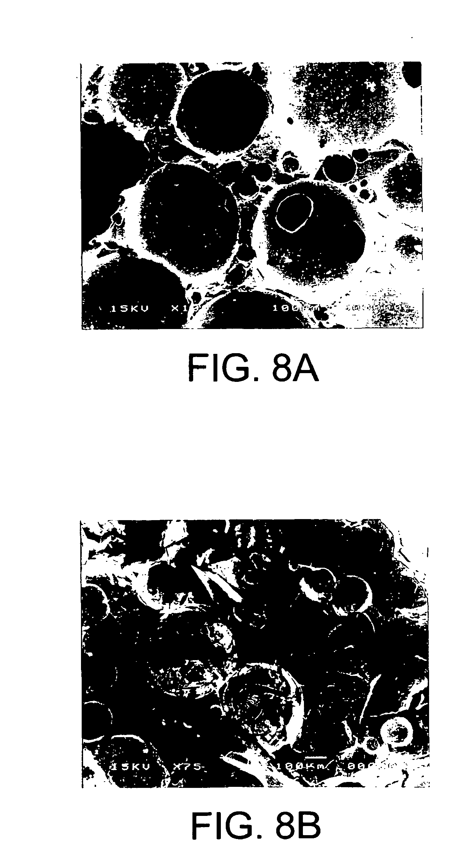

Figure 8A shows an SEM micrograph of a scaffold with a crack-free pore network, with spherical pores with diameters up to 600 µm, and interconnected pores of up to 100 µm in diameter. It is these interconnections that are vital for vascularization and tissue ingrowth. This scaffold was foamed at 25°C and created a sufficient foam volume (110 ml) to create such pores. Figure 8B shows an SEM micrograph of a scaffold with a cracked surface and fragmented pores, due to the low foam volumes achieved at higher temperatures (greater than 28°C) and faster gelling times.

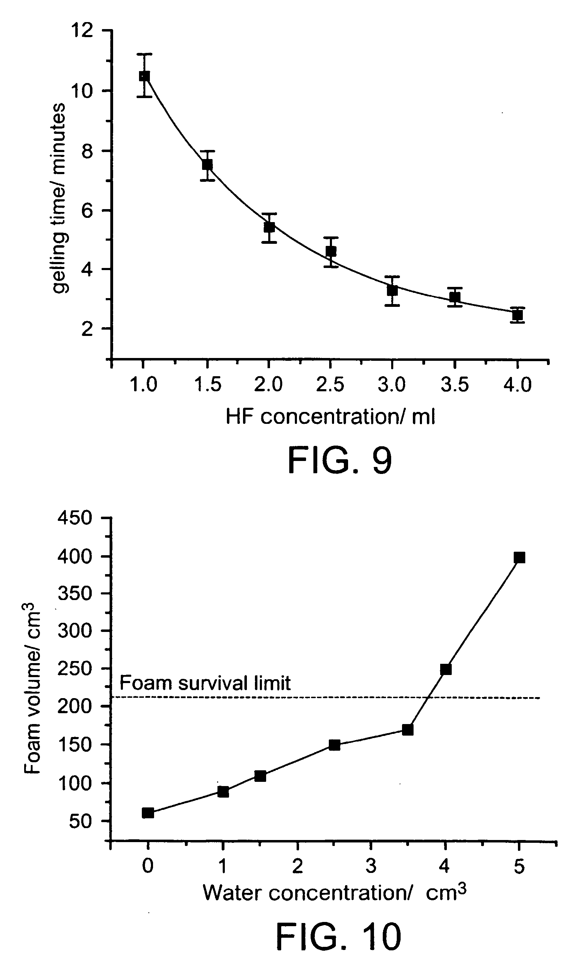

Figure 9 shows a graph of gelling time as a function of gelling agent (HF) concentration. The gelling time decreased as gelling agent concentration increased. However, foam volume was approximately constant as gelling time decreased.

Figure 10 shows a graph of foam volume as a function of water concentration added during the foaming process. This graph shows the unexpected result of the addition of a small amount of water to increase foam volume by greater than 2 times. As water concentration increased, foam volume unexpectedly increased, and therefore modal pore size increased.

The relationship between gelling time and foaming temperature can be fitted to a first order exponential decay (r² = 0.959). Hydrolysis is completed soon after mixing, therefore the condensation reaction is the rate-determining step for gelation. The condensation reaction is complex, but if the gelling time (t_{gel}) is the time over which viscosity has increased from approximately 10⁻¹P to 10⁴P then t_{gel} can be considered as an averaged rate of gelation. Therefore the exponential decay can be fitted to an Arrhenius equation: 1/t_{gel} = Aexp(-E*/RT_{f}), where A is an Arrhenius constant, R is the gas constant and E* is an apparent activation energy. It has, thus, been discovered that there is an apparent activation energy barrier that has to be overcome for the condensation reaction to be complete and for gelling to occur. The activation energy for gelation was calculated from the slope of a plot of In[t_{gel}] as a function of 1/T_{f} and was found to be 0.002 eV. As temperature is increased the kinetics of the condensation reaction is increased (more monomers come into contact with each other), reducing the gelling time. Activation energy for gelling is affected by the pH and composition of the sol. As foaming temperature increased, gelling time decreased, which meant that agitation time decreased, and therefore foam volume and macro-pore diameter decreased. At 25°C, gelling times were reproducible to within 20s and a modal pore size (~100mm) with potential for tissue ingrowth and vascularisation was attained.

Table 2 summarizes the results from nitrogen adsorption characterization of the foams. All foams yield a type IV of isotherm (not shown), which is indicative of mesoporous materials. The hysteresis loops between adsorption and desorption modes are typical for materials with cylindrical pores. Table 2 shows that the foams exhibited mesoporous textures with a modal pore diameter in the range 7-13 nm and that a change in temperature had little effect on the mesoporosity of the foams. However, the change in foaming temperature had a marked effect on the specific surface area of the foams, with surface area increasing from 150.7 m²g⁻¹ at 20°C to 192.7 m²g⁻¹ at 25°C to 454.4 m²g⁻¹ at 35°C.

**Table 2. Summary of nitrogen adsorption analysis on 58S scaffolds foamed at different temperatures.**

| Foaming temperature/ °C | Specific surface area (BET)/ m²g⁻¹ | C constant (BET) | Modal mesopore diameter/ nm |
|---|---|---|---|
| 20 | 150.7 | 82.7 | 8.3 |
| 25 | 192.7 | 194.4 | 12.4 |
| 35 | 454.4 | 217.9 | 7.8 |

The nitrogen sorption results combined with mercury porosimetry results indicate that the bioactive foams are 3D hierarchical structures of an interconnected macropore network in a matrix containing mesopores. The temperature at which the foam is produced affects the stability and morphology of this structure. Other factors in the foaming process that affect this structure are the composition and volume of the sol, the pH of the sol (catalyst type and concentration), the surfactant type and concentration and the temperature of the thermal stabilization process.

All the variables investigated are believed to have an influence on the porosity and structure of the foam scaffolds. Unexpectedly, the variable that produced the simplest control over pore size of the foams is the amount of water added to aid the surfactant. Changes in added water concentration may be used at constant temperature and constant concentrations of surfactant and gelling agent, to produce different pore networks at reproducible gelling times of a particular glass composition.

While the invention has been described with preferred embodiments, it is to be understood that variations and modifications may be resorted to as will be apparent to those skilled in the art. Such variations and modifications are to be considered within the purview and the scope of the claims appended hereto.

## Claims

1. A process for producing a foamed bioactive sol-gel with a hierarchical structure having macropores with a mean size of 10 to 500 micrometers and mesopores of 10 to 500 angstroms comprising:
a. hydrolyzing a reaction mixture comprising metal alkoxide capable of forming a bioactive sol-gel;
b. accelerating condensation of the reaction mixture by adding an acidic catalyst;
c. foaming the reaction mixture by adding a surfactant and vigorously agitating the reaction mixture;
d. casting the foamed reaction mixture into a mold of desired shape to complete formation of the foamed bioactive sol-gel; and
e. ageing, drying and thermally stabilizing the foamed bioactive sol-gel.

2. The process of claim 1, wherein the metal alkoxide comprise tetraethoxyorthosilicate, triethoxyphosphate or a combination thereof.

3. The process of claim 2, wherein the reaction mixture further comprises nitric acid, calcium nitrate, or a mixture thereof.

4. The process of claim 1, wherein the acidic catalyst is HF.

5. The process of claim 1, wherein the surfactant is a nonionic or anionic surfactant or mixtures thereof.

6. The process of claim 1, wherein water is added during foaming step c.

7. The process of claim 1, wherein the foamed bioactive sol-gel is aged and dried over a period of from 5 to 7 days.

8. The process of claim 4, wherein a temperature of 22 to 28°C is maintained during foaming.

9. The process of claim 1 further comprising carrying out step c until the reaction mixture begins to gel.

10. The process of claim 1 wherein the foamed bioactive sol-gel has macropores with a mean size of 50 to 250 micrometers and mesopores of 75 to 250 angstroms.

11. A biocompatible substrate for growing cells comprising a foamed bioactive sol-gel having a hierarchical structure, macropores with a mean size of 10 to 500 micrometers and mesopores in the range of 10 to 500 angstroms.

12. The biocompatible substrate of claim 11 further comprising a three-dimensional open network of spherical pores that are thoroughly interconnected.

13. The biocompatible substrate of claim 11 or claim 12 wherein the foamed sol- gel is formed from pure silica glass, binary SiO₂-CaO glass or ternary SiO₂- CaO-P₂0₅ glass.

14. The biocompatible substrate of any one of claims 11-13 wherein the foamed sol- gel is formed from binary Si0₂-CaO glass or ternary SiO₂-CaO-P₂0₅ glass.

15. The biocompatible substrate of any one of claims 11-14 wherein the growing cells are osteoblasts and the biocompatible substrate is used for a bone implant or bone graft material.

16. A biological filter comprising a bioactive foamed sol-gel as claimed in claim 12 and wherein the bioactive foamed sol-gel is formed from binary SiO₂-CaO glass or ternary Si0₂-CaO- P₂0₅ glass.

17. A drug delivery device comprising a bioactive foamed sol-gel as claimed in claim 12 and wherein the bioactive foamed sol-gel is formed from binary Si0₂-CaO glass or ternary Si0₂-CaO-P₂0₅ glass.

18. A foamed bioactive sol-gel made by the process of any one of claims 1 to 10.

19. A biocompatible substrate as claimed in claim 11, useful in tissue engineering structures, formed by first foaming a silica-based sol-gel in the presence of a catalyst and a surfactant with vigorous agitation at a controlled temperature of 22 to 28°C and then casting the foamed silica-based sol-gel into a mold of desired shape.

## Patentansprüche

1. Ein Verfahren zur Herstellung eines geschäumten, bioactiven Sol-Gels mit einer hierarchischen Struktur, die Makroporen einer mittleren Größe von 10 bis 500 Mikrometern und Mesoporen von 10 bis 500 Angström aufweist, bestehend aus:
a. Hydrolysieren einer Reaktionsmischung bestehend aus Metallalkoxid, das zur Bildung eines bioactiven Sol-Gels in der Lage ist;
b. Beschleunigung der Kondensation der Reaktionsmischung durch Zugabe eines sauren Katalysators;
c. Schäumen der Reaktionsmischung durch Zugabe eines Tensids und kräftiges Rühren der Reaktionsmischung;
d. Gießen der geschäumten Reaktionsmischung in eine Gießform gewünschter Form zur endgültigen Bildung des geschäumten, bioactiven Sol-Gels; und
e. Alterung, Trocknen und thermale Stabilisierung des geschäumten, bioactiven Sol-Gels.

2. Das Verfahren entsprechend Anspruch 1, wobei das Metallalkoxid Tetraethoxyorthosilicat, Triethoxyphosphat oder eine Kombination dieser enthält.

3. Das Verfahren entsprechend Anspruch 2, wobei die Reaktionsmischung zudem Salpetersäure, Calciumnitrat oder eine Mischung dieser enthält.

4. Das Verfahren entsprechend Anspruch 1, wobei der saure Katalysator HF ist.

5. Das Verfahren entsprechend Anspruch 1, wobei das Tensid ein nicht-ionisches oder anionisches Tensid oder eine Mischung dieser ist.

6. Das Verfahren entsprechend Anspruch 1, wobei während des Schäumens In Schritt c Wasser zugegeben wird.

7. Das Verfahren entsprechend Anspruch 1, wobei das geschäumte, bioactive Sol-Gel über einen Zeitraum von 5 bis 7 Tagen gealtert und getrocknet wird.

8. Das Verfahren entsprechend Anspruch 4, wobei während des Schäumens eine Temperatur von 22°C bis 28°C beibehalten wird.

9. Das Verfahren entsprechend Anspruch 1, zu dem weiterhin gehört, dass der Schritt c solange durchgeführt wird, bis die Reaktionsmischung zu gelleren beginnt.

10. Das Verfahren entsprechend Anspruch 1, wobei das geschäumte, bioactive Sol-Gel Makroporen einer mittleren Größe von 50 bis 250 Mikrometern und Mesoporen von 75 bis 250 Angström aufweist.

11. Ein biokompatibles Substrat zum Züchten von Zellen bestehend aus einem geschäumten, bioactiven Sol-Gel mit einer hierarchischen Struktur, Makroporen einer mittleren Größe von 10 bis 500 Mikrometern und Mesoporen von 10 bis 500 Angström.

12. Das blokompatible Substrat entsprechend Anspruch 11, das zudem aus einem dreidimensionalen, offenen Netz an kugelförmigen Poren besteht, die vollständig miteinander verknüpft sind.

13. Das blokompatible Substrat entsprechend Anspruch 11, wobei das geschäumte, bioactive Sol-Gel aus reinem Kieselglas, binärem SiO₂-CaO-Glas oder ternärem SiO₂-CaO-P₂O₅-Glas gebildet wird.

14. Das biokompatible Substrat entsprechend einem der Ansprüche 11-13, wobei das geschäumte, bioactive Sol-Gel aus binärem SiO₂-CaO-Glas oder ternärem SiO₂-CaO-P₂O₅-Glas gebildet wird.

15. Das biokompatible Substrat entsprechend einem der Ansprüche 11-14, wobei die wachsenden Zellen Osteoblasten sind und das biokompatible Substrat für ein Knochenimplantat oder Knochentransplantatsmaterial verwendet wird.

16. Ein biologischer Filter bestehend aus einem bioactlven, geschäumten Sol-Gel entsprechend Anspruch 12, und wobei das bioactive, geschäumte Sol-Gel aus binärem SiO₂-CaO-Glas oder ternärem SiO₂-CaO-P₂O₅-Glas gebildet wird.

17. Eine Vorrichtung zur Medikamentenverabreichung bestehend aus einem bioactiven, geschäumten Sol-Gel entsprechend Anspruch 12, und wobei das bioactive, geschäumte Sol-Gel aus binärem SiO₂-CaO-Glas oder ternärem SiO₂-CaO-P₂O₅-Glas gebildet wird.

18. Ein geschäumtes, bioactives Sol-Gel, das durch das Verfahren eines der Ansprüche 1 bis 10 hergestellt wird.

19. Ein biokompatibles Substrat entsprechend Anspruch 11, das In der Gewebekonstruktion/- züchtung nützlich ist, das durch zunächst Schäumen eines auf Kieselerde basierenden Sol-Gels in Gegenwart eines Katalysators und eines Tensid unter kräftigem Rühren bei einer kontrollierten Temperatur von 22°C bis 28°C gebildet wird, wonach das geschäumte Sol-Gel auf Kieselerdbasis in eine Grießform von gewünschter Form gegossen wird.

## Revendications

1. Procédé de fabrication d'un sol-gel bioactif expansé ayant une structure hiérarchique, des macropores d'une taille moyenne de 10 à 500 micromètres et des mésopores de 10 à 500 angstroms, comportant les étapes suivantes :
a. hydrolyse d'un mélange de réaction renfermant de l'alcoxyde métallique apte à former un sol-gel bioactif ;
b. accélération de la condensation du mélange de réaction par l'addition d'un catalyseur acide ;
c. expansion du mélange de réaction par l'addition d'un surfactant et par l'agitation vigoureuse du mélange de réaction ;
d. coulée du mélange de réaction expansé dans un moule de la forme souhaitée pour terminer la formation du sol-gel bioactif expansé ; et
e. vieillissement, séchage et stabilisation thermique du sol-gel bioactif expansé.

2. Procédé de la revendication 1, dans lequel l'alcoxyde métallique comprend du tétraéthoxyorthosilicate, du triéthoxyphosphate ou une combinaison de ceux-ci.

3. Procédé de la revendication 2, dans lequel le mélange de réaction comprend en outre de l'acide nitrique, du nitrate de calcium ou un mélange de ceux-ci.

4. Procédé de la revendication 1, dans lequel le catalyseur acide est du HF.

5. Procédé de la revendication 1, dans lequel le surfactant est un surfactant nonionique ou anionique ou des mélanges de ceux-ci.

6. Procédé de la revendication 1, dans lequel de l'eau est ajoutée pendant l'étape d'expansion c.

7. Procédé de la revendication 1, dans lequel le sol-gel bioactif est vieilli et séché pendant une période de 5 à 7 jours.

8. Procédé de la revendication 4, dans lequel une température de 22 à 28°C est maintenue pendant l'expansion.

9. Procédé de la revendication 1 comprenant en outre la réalisation de l'étape c jusqu'à ce que le mélange de réaction commence à se gélifier.

10. Procédé de la revendication 1 dans lequel le sol-gel biactif expansé a des macropores d'une taille moyenne de 50 à 250 micromètres et des mésopores de 75 à 250 angstroms.

11. Substrat biocompatible destiné à la croissance de cellules comprenant un sol-gel bioactif expansé ayant une structure hiérarchique, des macropores d'une taille moyenne de 10 à 500 micromètres et des mésopores se situant dans la plage de 10 à 500 angstroms.

12. Substrat biocompatible de la revendication 11 comprenant un réseau ouvert tridimensionnel de pores sphériques qui sont complètement interconnectés.

13. Substrat biocompatible de la revendication 11 ou de la revendication 12, dans lequel le sol-gel expansé est constitué de verre de silice pure, de verre SiO₂-CaO binaire ou de verre SiO₂-CaO-P₂0₅ ternaire.

14. Substrat biocompatible de l'une quelconque des revendications 11 à 13, dans lequel le sol-gel expansé est constitué de verre SiO₂-CaO binaire ou de verre SiO₂-CaO-P₂0₅ ternaire.

15. Substrat biocompatible de l'une quelconque des revendications 11 à 14, dans lequel les cellules en développement sont des ostéoblastes et le substrat biocompatible est utilisé pour un implant osseux ou un matériau de greffe osseuse.

16. Filtre biologique comprenant un sol-gel bioactif expansé selon la revendication 12 et dans lequel le sol-gel bioactif expansé est constitué de verre SiO₂-CaO binaire ou de verre SiO₂-CaO-P₂0₅ ternaire.

17. Dispositif d'administration de médicament comprenant un sol-gel biactif expansé selon la revendication 12 et dans lequel le sol-gel biactif expansé est constitué de verre SiO₂-CaO binaire ou de verre SiO₂-CaO-P₂-O₅ ternaire.

18. Sol-gel bioactif expansé fabriqué par le procédé de l'une quelconque des revendications 1 à 10.

19. Substrat biocompatible selon la revendication 11, utile dans les structures destinées au génie tissulaire, formé par l'expansion en premier lieu d'un sol-gel à base de silice en présence d'un catalyseur et d'un surfactant avec une agitation vigoureuse à une température contrôlée de 22 à 28°C, suivie de la coulée du sol-gel à base de silice expansé dans un moule de la forme souhaitée.
